# EUROPEAN PATENT APPLICATION

(11) **EP 4 835 900 A1**
(43) Date of publication of application: **04.11.2026**
(21) Application number: 24912842.2
(22) Date of filing: 24.12.2024
(51) Int. Cl.: G16C 60/00, G06N 99/00

(54) **INFORMATION PROCESSING DEVICE, INFORMATION PROCESSING SYSTEM, PROGRAM, AND METHOD FOR ASSISTING WITH CREATION OF ISING MODEL**

(30) Priority: 28.12.2023 JP 2023222639; 05.03.2024 JP 2024033063
(71) Applicant: Resonac Corporation, Tokyo 105-7325 (JP)
(72) Inventor: KAKUDA, Kohsuke, Tokyo 105-7325 (JP); SAKAGUCHI, Suguru, Tokyo 105-7325 (JP); OKUNO, Yoshishige, Tokyo 105-7325 (JP)
(74) Representative: Strehl & Partner mbB
(86) International application number: PCT/JP2024/045754
(87) International publication number: WO 2025/142956

(57) **Abstract**

An information processing device configured to assist preparation of an Ising model for allowing an annealing-type optimization machine to solve an optimal solution search problem for a composite material with constraints on proportions or contents in a composition of the composite material, the information processing device including:
an input receiving part configured to receive as input:
an objective function that represents a property of the composite material according to the proportions or contents in the composition of the composite material; and
a constraint on the proportions or contents in the composition of the composite material; and

a data preparation part configured to prepare the Ising model that allows the annealing-type optimization machine to find an optimal solution for the proportions or contents in the composition of the composite material, the optimal solution satisfying a constraint expression that represents the constraint on the proportions or contents in the composition of the composite material and optimizing the property of the composite material,
in which, when the composite material is a mixture of substances:
a quantity rᵢ of a substance i is indicated by a bit representation; and
whether or not the substance i is contained in the composite material is indicated by the bit representation,

in which the constraint expression includes conditions that require that following mathematical expressions 1 and 2 be satisfied: [Math 1] $\left\{\begin{matrix}\left({m}_{B}+1\right) \left(1-{q}_{A 0}\right) + {\sum }_{j = 1}^{{m}_{i}} {q}_{Bj} \leq {m}_{B} + 1 & \left(1\right) \\ \left({m}_{B}+1\right) \left(1-{q}_{A 0}\right) + {\sum }_{j = 1}^{{m}_{i}} {q}_{Bj} \geq 1 - {q}_{B 0} & \left(2\right)\end{matrix}\right. ,$ where:
q_{A0} is an auxiliary variable that is 0 when a substance A is contained in the composite material and that is 1 when the substance A is not contained in the composite material;
q_{B0} is an auxiliary variable that is 0 when a substance B is contained in the composite material and that is 1 when the substance B is not contained in the composite material;
m_{B} is a number of bits required to represent a quantity r_{B} of the substance B to be mixed in the composite material; and
q_{Bj} is a number 0 or 1 of a binary representation of a proportion or content of the substance B, and
in which the constraint expression is arranged such that, when a condition included in the constraint expression fails to satisfy at least one of the mathematical expression 1 or 2, a proportion or content represented by the failing condition is excluded from the optimal solution for the composition of the composite material.

## Description

### Technical Field

The present disclosure relates to an information processing device, an information processing system, a program, and a method of assisting preparation of Ising models.

### Background Art

A technique for finding a stable combination of a site A, a site B, and an anion site in a Perovskite-type crystal structure at a high speed, even when there are enormous potential combinations, by performing a ground-state search using simulated annealing with Ising models or quadratic unconstrained binary optimization (QUBO), has been known heretofore (see, for example, Patent Document 1).

### Citation List

### Patent Documents

Patent Document 1: Japanese Patent Application Publication No. 2021-033768

### Summary of Invention

### Technical Problem

An optimal solution search problem is used, for example, when selecting an optimal combination of proportions or contents in composition from many potential combinations, such as when searching for a composition in which a composite material's properties are optimized. An annealing-type optimization machine can solve optimal solution search problems that are formulated based on the Ising model.

In the event an optimal combination of proportions or contents in a composition is to be selected from many potential combinations, such as when searching for a composition in which a composite material's properties are optimized, the composition of the target composite material is subject to various constraints in many cases. Furthermore, since there is a risk that substances that are mutually incompatible may be combined together, constraints on these substances' combinations may be applied as constraints on proportions or contents in the composition of the target composite material. The Ising model allows for imposition of constraints on proportions or contents in composition.

According to the Ising model, for example, a constraint (penalty function) may serve to prevent a combination of proportions or contents in a composition that does not satisfy the constraint on proportions or contents in the composition from being selected as an optimal combination. However, although constraints need to be provided in Ising mathematical expressions (constraint terms), transforming constraints on a composition into constraint terms has heretofore been difficult.

The present disclosure therefore aims to provide an information processing device, an information processing system, a program, and a method for assisting preparation of Ising models that will allow an annealing-type optimization machine to solve an optimal solution search problem for a composite material with constraints on proportions or contents in its composition.

### Solution to Problem

The present disclosure may be carried out, for example, as follows:
[1] An information processing device configured to assist preparation of an Ising model for allowing an annealing-type optimization machine to solve an optimal solution search problem for a composite material with constraints on proportions or contents in a composition of the composite material, the information processing device including:
   an input receiving part configured to receive as input:
      an objective function that represents a property of the composite material according to the proportions or contents in the composition of the composite material; and
      a constraint on the proportions or contents in the composition of the composite material; and
   a data preparation part configured to prepare the Ising model that allows the annealing-type optimization machine to find an optimal solution for the proportions or contents in the composition of the composite material, the optimal solution satisfying a constraint expression that represents the constraint on the proportions or contents in the composition of the composite material and optimizing the property of the composite material,
   in which, when the composite material is a mixture of substances:
      a quantity rᵢ of a substance i is indicated by a bit representation; and
      whether or not the substance i is contained in the composite material is indicated by the bit representation,
   in which the constraint expression includes conditions that require that following mathematical expressions 1 and 2 be satisfied:
      [Math 1] $\left\{\begin{matrix}\left({m}_{B}+1\right) \left(1-{q}_{A 0}\right) + {\sum }_{j = 1}^{{m}_{i}} {q}_{Bj} \leq {m}_{B} + 1 & \left(1\right) \\ \left({m}_{B}+1\right) \left(1-{q}_{A 0}\right) + {\sum }_{j = 1}^{{m}_{i}} {q}_{Bj} \geq 1 - {q}_{B 0} & \left(2\right)\end{matrix}\right. ,$ where:
      q_{A0} is an auxiliary variable that is 0 when a substance A is contained in the composite material and that is 1 when the substance A is not contained in the composite material;
      q_{B0} is an auxiliary variable that is 0 when a substance B is contained in the composite material and that is 1 when the substance B is not contained in the composite material;
      m_{B} is a number of bits required to represent a quantity r_{B} of the substance B to be mixed in the composite material; and
      q_{Bj} is a number 0 or 1 of a binary representation of a proportion or content of the substance B, and
      in which the constraint expression is arranged such that, when a condition included in the constraint expression fails to satisfy at least one of the mathematical expression 1 or 2, a proportion or content represented by the failing condition is excluded from the optimal solution for the composition of the composite material.
[2] The information processing device according to [1], in which the constraint expression further includes conditions that require that following mathematical expressions 3 and 4 be satisfied:
   [Math 2] $\left\{\begin{matrix}\left({m}_{A}+1\right) \left(1-{q}_{B 0}\right) + {\sum }_{j = 1}^{{m}_{i}} {q}_{Aj} \leq {m}_{A} + 1 & \left(3\right) \\ \left({m}_{A}+1\right) \left(1-{q}_{B 0}\right) + {\sum }_{j = 1}^{{m}_{i}} {q}_{Aj} \geq 1 - {q}_{A 0} & \left(4\right)\end{matrix}\right. ,$ where:
   q_{A0} is the auxiliary variable that is 0 when the substance A is contained in the composite material and that is 1 when the substance A is not contained in the composite material;
   q_{B0} is the auxiliary variable that is 0 when the substance B is contained in the composite material and that is 1 when the substance B is not contained in the composite material;
   m_{A} is a number of bits required to represent a quantity r_{A} of the substance A to be mixed in the composite material; and
   g_{Aj} is a number 0 or 1 of a binary representation of a proportion or content of the substance A in the composite material, and
   in which the constraint expression is arranged such that, when a condition included in the constraint expression fails to satisfy at least one of the mathematical expression 3 or 4, a proportion or content represented by the failing condition is excluded from the optimal solution for the composition of the composite material.
[3] The information processing device according to [1] or [2], in which, when a condition regarding a quantity of a substance to be mixed in the composite material is representation by a continuous number:
   the quantity rᵢ of the substance i is indicated by a bit representation of a following mathematical expression 5; and
   whether or not the substance i is contained in the composite material is indicated by the bit representation of the mathematical expression 5:
      [Math 3] ${r}_{i} = {\sum }_{j = 1}^{{m}_{i}} {C}_{j} {q}_{ij}$ (5), where:
      qᵢⱼ is a number 0 or 1 of a binary representation of a proportion or content of the substance i;
      Cⱼ is a coefficient related to the binary representation of the proportion or content of the substance i;
      C₁ is a minimum unit for the quantity of substances to be mixed in the composite material;
      mᵢ is a maximum natural number at which Cₘ < M_{i,MAX} holds true; and
      M_{i,MAX} is a maximum quantity for the quantity rᵢ of the substance i.
[4] The information processing device according to one of [1] to [3], in which, when a condition regarding a quantity of a substance to be mixed in the composite material is representation by a discrete number:
   the quantity rᵢ of the substance i is indicated by the bit representation; and
   whether or not the substance i is contained in the composite material is indicated by the bit representation.
[5] The information processing device according to [1] to [4], in which the mathematical expressions 1 and 2 represent constraints on the proportions or contents in the composition of the composite material specifying that the substance B is not used when the substance A is used.
[6] The information processing device according to [2], in which the mathematical expressions 3 and 4 represent constraints on the proportions or contents in the composition of the composite material specifying that the substance A is not used when the substance B is used.
[7] An information processing system including:
   an annealing-type optimization machine; and
   an information processing device configured to assist preparation of an Ising model for allowing the annealing-type optimization machine to solve an optimal solution search problem for a composite material with constraints on proportions or contents in a composition of the composite material,
   in which the information processing device includes:
      an input receiving part configured to receive as input:
         an objective function that represents a property of the composite material according to the proportions or contents in the composition of the composite material; and
         a constraint on the proportions or contents in the composition of the composite material; and
      a data preparation part configured to prepare the Ising model that allows the annealing-type optimization machine to find an optimal solution for the proportions or contents in the composition of the composite material, the optimal solution satisfying a constraint expression that represents the constraint on the proportions or contents in the composition of the composite material and optimizing the property of the composite material,
      in which, when the composite material is a mixture of substances:
         a quantity rᵢ of a substance i is indicated by a bit representation; and
         whether or not the substance i is contained in the composite material is indicated by the bit representation,
      in which the constraint expression includes conditions that require that following mathematical expressions 1 and 2 be satisfied:
         [Math 4] $\left\{\begin{matrix}\left({m}_{B}+1\right) \left(1-{q}_{A 0}\right) + {\sum }_{j = 1}^{{m}_{i}} {q}_{Bj} \leq {m}_{B} + 1 & \left(1\right) \\ \left({m}_{B}+1\right) \left(1-{q}_{A 0}\right) + {\sum }_{j = 1}^{{m}_{i}} {q}_{Bj} \geq 1 - {q}_{B 0} & \left(2\right)\end{matrix}\right. ,$ where:
         q_{A0} is an auxiliary variable that is 0 when a substance A is contained in the composite material and that is 1 when the substance A is not contained in the composite material;
         q_{B0} is an auxiliary variable that is 0 when a substance B is contained in the composite material and that is 1 when the substance B is not contained in the composite material;
         m_{B} is a number of bits required to represent a quantity r_{B} of the substance B to be mixed in the composite material; and
         q_{Bj} is a number 0 or 1 of a binary representation of a proportion or content of the substance B in the composite material, and
         in which the constraint expression is arranged such that, when a condition included in the constraint expression fails to satisfy at least one of the mathematical expression 1 or 2, a proportion or content represented by the failing condition is excluded from the optimal solution for the composition of the composite material.
[8] A program for causing an information processing device to perform steps to assist preparation of an Ising model for allowing an annealing-type optimization machine to solve an optimal solution search problem for a composite material with constraints on proportions or contents in a composition of the composite material, the steps including:
   receiving as input:
      an objective function that represents a property of the composite material according to the proportions or contents in the composition of the composite material; and
      a constraint on the proportions or contents in the composition of the composite material; and
   preparing the Ising model that allows the annealing-type optimization machine to find an optimal solution for the proportions or contents in the composition of the composite material, the optimal solution satisfying a constraint expression that represents the constraint on the proportions or contents in the composition of the composite material and optimizing the property of the composite material,
   in which, when the composite material is a mixture of substances:
      a quantity rᵢ of a substance i is indicated by a bit representation; and
      whether or not the substance i is contained in the composite material is indicated by the bit representation,
   in which the constraint expression includes conditions that require that following mathematical expressions 1 and 2 be satisfied:
      [Math 5] $\left\{\begin{matrix}\left({m}_{B}+1\right) \left(1-{q}_{A 0}\right) + {\sum }_{j = 1}^{{m}_{i}} {q}_{Bj} \leq {m}_{B} + 1 & \left(1\right) \\ \left({m}_{B}+1\right) \left(1-{q}_{A 0}\right) + {\sum }_{j = 1}^{{m}_{i}} {q}_{Bj} \geq 1 - {q}_{B 0} & \left(2\right)\end{matrix}\right. ,$ where:
      q_{A0} is an auxiliary variable that is 0 when a substance A is contained in the composite material and that is 1 when the substance A is not contained in the composite material;
      q_{B0} is an auxiliary variable that is 0 when a substance B is contained in the composite material and that is 1 when the substance B is not contained in the composite material;
      m_{B} is a number of bits required to represent a quantity r_{B} of the substance B to be mixed in the composite material; and
      q_{Bj} is a number 0 or 1 of a binary representation of a proportion or content of the substance B, and
      in which the constraint expression is arranged such that, when a condition included in the constraint expression fails to satisfy at least one of the mathematical expression 1 or 2, a proportion or content represented by the failing condition is excluded from the optimal solution for the composition of the composite material.
[9] A method for assisting preparation of an Ising model for allowing an annealing-type optimization machine to solve an optimal solution search problem for a composite material with constraints on proportions or contents in a composition of the composite material, the method including:
   receiving as input:
      an objective function that represents a property of the composite material according to the proportions or contents in the composition of the composite material; and
      a constraint on the proportions or contents in the composition of the composite material; and
   preparing the Ising model that allows the annealing-type optimization machine to find an optimal solution for the proportions or contents in the composition of the composite material, the optimal solution satisfying a constraint expression that represents the constraint on the proportions or contents in the composition of the composite material and optimizing the property of the composite material,
   in which, when the composite material is a mixture of substances:
      a quantity rᵢ of a substance i is indicated by a bit representation; and
      whether or not the substance i is contained in the composite material is indicated by the bit representation,
   in which the constraint expression includes conditions that require that following mathematical expressions 1 and 2 be satisfied:
      [Math 6] $\left\{\begin{matrix}\left({m}_{B}+1\right) \left(1-{q}_{A 0}\right) + {\sum }_{j = 1}^{{m}_{i}} {q}_{Bj} \leq {m}_{B} + 1 & \left(1\right) \\ \left({m}_{B}+1\right) \left(1-{q}_{A 0}\right) + {\sum }_{j = 1}^{{m}_{i}} {q}_{Bj} \geq 1 - {q}_{B 0} & \left(2\right)\end{matrix}\right. ,$ where:
      q_{A0} is an auxiliary variable that is 0 when a substance A is contained in the composite material and that is 1 when the substance A is not contained in the composite material;
      q_{B0} is an auxiliary variable that is 0 when a substance B is contained in the composite material and that is 1 when the substance B is not contained in the composite material;
      m_{B} is a number of bits required to represent a quantity r_{B} of the substance B to be mixed in the composite material; and
      q_{Bj} is a number 0 or 1 of a binary representation of a proportion or content of the substance B, and
      in which the constraint expression is arranged such that, when a condition included in the constraint expression fails to satisfy at least one of the mathematical expression 1 or 2, a proportion or content represented by the failing condition is excluded from the optimal solution for the composition of the composite material.
[10] An information processing device configured to assist preparation of an Ising model for allowing an annealing-type optimization machine to solve an optimal solution search problem for a composite material with constraints on proportions or contents in a composition of the composite material, the information processing device including:
   an input receiving part configured to receive as input:
      an objective function that represents a property of the composite material according to the proportions or contents in the composition of the composite material; and
      a constraint on the proportions or contents in the composition of the composite material; and
   a data preparation part configured to prepare the Ising model that allows the annealing-type optimization machine to find an optimal solution for the proportions or contents in the composition of the composite material, the optimal solution satisfying a constraint expression that represents the constraint on the proportions or contents in the composition of the composite material and optimizing the property of the composite material,
   in which, when the composite material is a mixture of substances:
      a quantity rᵢ of a substance i is indicated by a bit representation; and
      whether or not the substance i is contained in the composite material is indicated by the bit representation,
   in which the constraint expression includes conditions that require that following mathematical expressions 6, 7, and 8 be satisfied:
      [Math 7] $\left\{\begin{matrix}\left({m}_{i}+1\right) {q}_{i 0} + {\sum }_{j = 1}^{{m}_{i}} {q}_{ij} \leq {m}_{i} + 1 & \left(6\right) \\ \left({m}_{i}+1\right) {q}_{i 0} + {\sum }_{j = 1}^{{m}_{i}} {q}_{ij} \geq 1 & \left(7\right) \\ {\sum }_{i = 1}^{N} {q}_{i 0} \geq N - 1 & \left(8\right)\end{matrix}\right. ,$ where:
      qᵢ₀ is an auxiliary variable that is 0 when the substance i is contained in the composite material and that is 1 when the substance i is not contained in the composite material;
      mᵢ is a number of bits required to represent the quantity rᵢ of the substance i to be mixed in the composite material; and
      qᵢⱼ is a number 0 or 1 of a binary representation of a proportion or content of the substance i in the composite material, and
      N is a number of substances i subject to a constraint represented by the mathematical expression 8, and
      in which the constraint expression is arranged such that, when a condition included in the constraint expression fails to satisfy at least one of the mathematical expression 6, 7, or 8, a proportion or content represented by the failing condition is excluded from the optimal solution for the composition of the composite material.

### Advantageous Effects of the Invention

The present disclosure thus provides an information processing device, an information processing system, a program, and a method for assisting preparation of Ising models that will allow an annealing-type optimization machine to solve an optimal solution search problem for a composite material with constraints on proportions or contents in its composition.

### Brief Description of Drawings

[FIG. 1] FIG. 1 is a diagram showing an example architecture of an information processing system according to an embodiment of the present disclosure (hereinafter "the present embodiment");
[FIG. 2] FIG. 2 is a diagram showing an example hardware configuration of a computer according to the present embodiment;
[FIG. 3] FIG. 3 is a diagram for explaining examples of constraints on a composition;
[FIG. 4] FIG. 4 is a diagram for explaining examples of constraints on a composition;
[FIG. 5] FIG. 5 is a diagram showing an example architecture of an information processing system according to the present embodiment;
[FIG. 6] FIG. 6 is a diagram for explaining examples of bit representations related to a substance;
[FIG. 7A] FIG. 7A is a diagram for explaining bit representations related to a substance 1 with specific examples;
[FIG. 7B] FIG. 7B is another diagram for explaining bit representations related to substance 1 with specific examples;
[FIG. 7C] FIG. 7C is another diagram for explaining bit representations related to substance 1 with specific examples;
[FIG. 8] FIG. 8 is a diagram for explaining examples of bit representations related to a substance;
[FIG. 9] FIG. 9 is a diagram for explaining examples of bit representations related to a composite material's composition;
[FIG. 10A] FIG. 10A is a diagram for explaining constraints represented by a mathematical expression 12 and a mathematical expression 13 with examples;
[FIG. 10B] FIG. 10B is a diagram for explaining constraints represented by mathematical expressions 12 and 13 with examples;
[FIG. 11A] FIG. 11A is a diagram for explaining constraints represented by a mathematical expression 14 and a mathematical expression 15 with examples;
[FIG. 11B] FIG. 11B is a diagram for explaining constraints represented by mathematical expressions 14 and 15 with examples;
[FIG. 12A] FIG. 12A is a diagram for explaining an example usage of constraints represented by mathematical expressions 14 and 15;
[FIG. 12B] FIG. 12B is a diagram for explaining an example usage of constraints represented by mathematical expressions 14 and 15;
[FIG. 12C] FIG. 12C is a diagram for explaining an example usage of constraints represented by mathematical expressions 14 and 15;
[FIG. 12D] FIG. 12D is a diagram for explaining an example usage of constraints represented by mathematical expressions 14 and 15;
[FIG. 13A] FIG. 13A is a diagram for explaining an example usage of constraints represented by mathematical expressions 14 and 15, and constraints represented by a mathematical expression 16 and a mathematical expression 17;
[FIG. 13B] FIG. 13B is a diagram for explaining an example usage of constraints based on mathematical expressions 14 and 15 and constraints based on mathematical expressions 16 and 17;
[FIG. 14] FIG. 14 is a flowchart showing examples of processing steps performed by an information processing system according to the present embodiment;
[FIG. 15] FIG. 15 is a diagram for explaining example usages of a constraint represented by a mathematical expression 18; and
[FIG. 16] FIG. 16 is a diagram for explaining example usages of constraints based on mathematical expression 18.

### Description of Embodiments

Next, an embodiment of the present invention will be described in detail. However, the present invention is by no means limited to the following embodiment alone.

### <System Architecture>

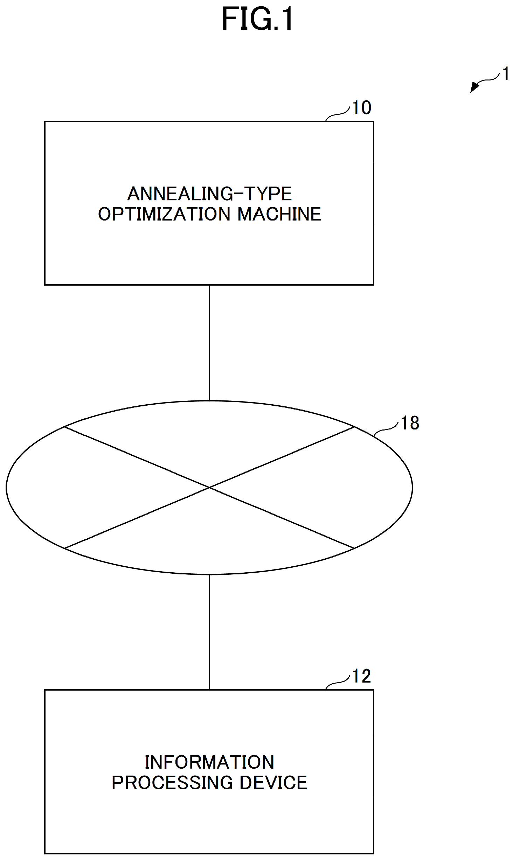

FIG. 1 is a diagram showing an example architecture of an information processing system according to the present embodiment. The information processing system 1 shown in FIG. 1 includes at least an annealing-type optimization machine 10 and an information processing device 12. The annealing-type optimization machine 10 and the information processing device 12 are connected such that data can be communicated between them via a communication network 18, which may be a local area network (LAN), the Internet, or the like.

The annealing-type optimization machine 10 is an example of a device that solves optimal solution search problems (also "optimization problems") using Ising models. An optimization problem refers to a problem for finding a solution that minimizes or maximizes an objective function among solutions that satisfy a given constraint. In an optimization problem, the solution to be found is expressed using decision variables. The objective function is a function in which a value to be minimized or maximized is expressed using decision variables. A constraint is a relational expression in which a requirement that must be satisfied is expressed using decision variables.

Furthermore, a combination optimization problem is an optimization problem having a combinatorial structure. That is, a combination optimization problem is one in which, for example, among combinations of decision variables that satisfy a constraint, a combination of decision variables that minimizes or maximizes the objective function is determined.

The annealing-type optimization machine 10 may be implemented by using a quantum computer that supports a quantum annealing system, or by using an Ising machine (annealing machine) that supports a quantum annealing system with digital circuitry such as a field programmable gate array (FPGA), a graphics processing unit (GPU), etc. The annealing-type optimization machine 10 may be implemented, for example, using a digital annealer (registered trademark), which is an example of an Ising machine.

The annealing-type optimization machine 10, for example, reduces an optimization problem to Ising models and finds a solution based on the Ising models' converging operation. Note that an Ising model can be expressed using QUBO. An Ising model's energy function and a QUBO cost function are equivalent through variable transformation.

The Ising model is a statistical model of a magnetic substance's behavior. The Ising model is characterized in that the state of a spinning magnetic substance is updated such that reciprocal interactions between the magnetic substance's spins lead to minimization of energy (Hamiltonian), ultimately resulting in minimum energy. The annealing-type optimization machine 10 reduces an optimization problem to Ising models and determines in what state the energy is minimized, finding this state as an optimal solution for the optimization problem.

The information processing device 12 is a user-operated device such as a personal computer (PC), a tablet terminal, a smartphone, and the like. Assuming that a user wants the annealing-type optimization machine 10 to solve an optimization problem, how the information processing device 12 then assists or helps the user prepare Ising models that will allow the annealing-type optimization machine 10 to solve the optimization problem will be as described below.

The information processing device 12 also prepares input information for the annealing-type optimization machine 10 based on user operations. The input information is entered in the annealing-type optimization machine 10 to allow the annealing-type optimization machine 10 to solve the optimization problem. The input information to be entered in the annealing-type optimization machine 10 includes, for example, objective functions and constraints written in Ising form, which also will be described below.

By entering input information prepared thus for the annealing-type optimization machine 10 in the annealing-type optimization machine 10, the user can have the annealing-type optimization machine 10 solve the optimization problem having been reduced to Ising models.

In this way, the information processing device 12 assists the user in preparing Ising models that will allow the annealing-type optimization machine 10 to solve an optimization problem. The information processing device 12 receives the optimal solution the annealing-type optimization machine 10 finds with respect to the optimization problem and outputs this optimal solution in a way that allows the user to check it. For example, the information processing device 12 may show the optimal solution on a display device.

Note that the information processing system 1 in FIG. 1 is one example. The information processing system 1 may also be configured such that, for example, a user accesses and uses the information processing device 12 from a user terminal (not shown) connected to the information processing device 12 via the communication network 18.

The annealing-type optimization machine 10 may be implemented as a cloud computing service. For example, the annealing-type optimization machine 10 may be available by calling an application programming interface (API) via the communication network 18.

Furthermore, the annealing-type optimization machine 10 need not be implemented as a cloud computing service, and may be implemented on premises, may be run and provided by a different/remote company, and so forth. The annealing-type optimization machine 10 may also be implemented using multiple computers.

Furthermore, when a user accesses and uses an information processing device 12, the information processing device 12 may be implemented as a cloud computing service, may be implemented on premises, may be run and provided by a different/remote company, or may be implemented using multiple computers. That is, the information processing system 1 of FIG. 1 is available in a variety of architectures depending on the manner and purpose of use, for example.

### <Hardware Configuration>

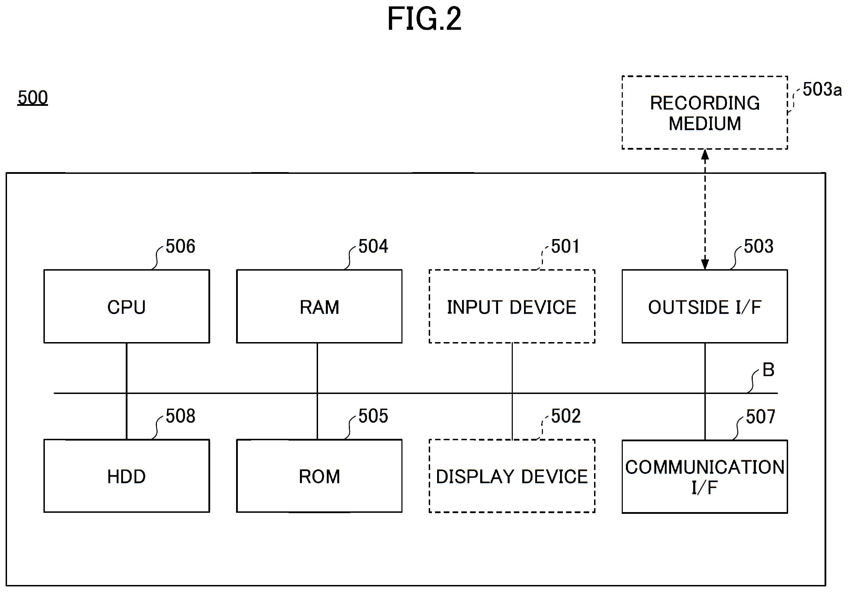

The information processing device 12 of FIG. 1 is implemented, for example, by a computer 500 having the hardware configuration shown in FIG. 2.

FIG. 2 is a diagram showing an example hardware configuration of a computer according to the present embodiment. The computer 500 in FIG. 2 includes at least an input device 501, a display device 502, an outside interface ("I/F") 503, a RAM 504, a ROM 505, a CPU 506, a communication I/F 507, and a HDD 508, all of which are mutually connected via buses B. An example in which the input device 501 and the display device 502 are connected via the communication I/F 507 to the computer 500 is also applicable.

The input device 501 is a touch panel, touch keys, buttons, keyboard, mouse, etc., used by the user to input various signals. The display device 502 is comprised of a display such as an LCD or OLED that displays the screen, a speaker that outputs audio data such as sound and voice, etc. The communication I/F 507 is an interface through which the computer 500 communicates data.

The HDD 508 is an example of a non-volatile storage device that stores programs, data, etc. The programs and data stored in the HDD 508 include the OS, which is the basic software that controls the entire computer 500, applications that enable various functions on the OS, and so forth. Instead of the HDD 508, the computer 500 may use a drive device that uses a flash memory as a storage medium (for example, a solid-state drive (SSD)).

The communication I/F 503 is an interface with outside devices. Examples of outside devices include a recording medium 503a, for example. This allows the computer 500 to read and/or write data to the recording medium 503a via the communication I/F 503. The recording medium 503a may be flexible disk, a CD, a DVD, an SD memory card, a USB memory, and so forth.

The ROM 505 is an example of a non-volatile semiconductor memory (storage device) that can hold programs and data even when the computer 500 is powered off. The ROM 505 stores programs and data such as the BIOS, OS configuration, and network configuration that are executed when the computer 500 is started. The RAM 504 is an example of a volatile semiconductor memory (storage device) that holds programs and data on a temporary basis.

The CPU 506 is a computing device that controls the entire computer 500 and implements its functions by loading programs and data from storage devices such as the ROM 505 and the HDD 508, onto the RAM 504, and executing processes. The information processing device 12 of the present embodiment can implement various functions, which will be described later. The hardware configuration of the annealing-type optimization machine 10 will not be described.

### <Examples of Problems Solved as Optimization Problems>

Assuming there is a composite material with constraints on proportions or contents in its composition, an example of solving a combination optimization problem by selecting a composition in which the composite material's properties are optimized will be described below.

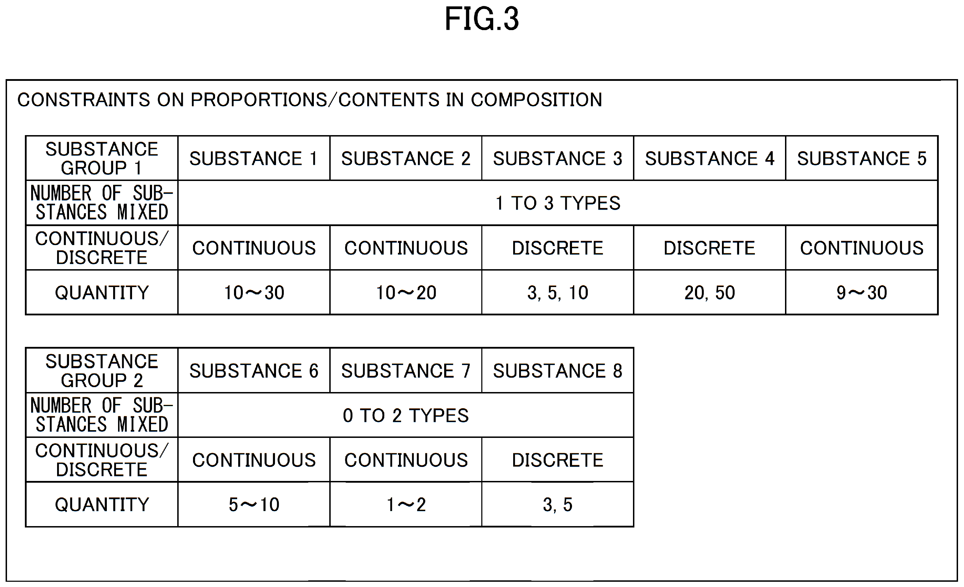

For example, as shown in FIG. 3, the present embodiment imposes constraints on a composition. FIG. 3 is a diagram for explaining examples of constraints on a composition. A composite material is comprised of multiple substance groups. In the example of FIG. 3, a composite material is made up of a substance group 1 and a substance group 2. Furthermore, in the example illustrated in FIG. 3, every substance constituting at least part of the composite material is subject to constraints on a composition.

The constraints on a composition in FIG. 3 include items such as "the number of substances mixed," "continuous/discrete," and "quantity." "The number of substances mixed" is a condition that indicates how many substances among the substances included in each substance group are to be mixed in the target composite material. The minimum and maximum number of substances to be mixed are set. "Continuous/discrete" is a condition that indicates the quantity of each substance to be mixed in the target composite material and whether the quantity of each substance to be mixed in the composite material is represented by a continuous number or a discrete number.

"Quantity" is a condition that indicates the quantity of each substance to be mixed in the composite material. When the quantity of a substance to be mixed in a composite material is represented by a continuous number, a minimum quantity and a maximum quantity are set in "Quantity" for that substance. When the quantity of a substance to be mixed in a composite material is a discrete number, the quantity of that substance is set in "Quantity."

For example, the constraints on a composition set forth in FIG. 3 show that: a substance group 1 contains five substances, namely a substance 1 to a substance 5; and, among the five substances 1 to 5, one to three substances are mixed in the composite material. Furthermore, the constraints on a composition set forth in FIG. 3 show that: a substance group 2 contains three substances, namely a substance 6 to a substance 8; and, among the three substances 6 to 8, zero to two substances are mixed in the composite material.

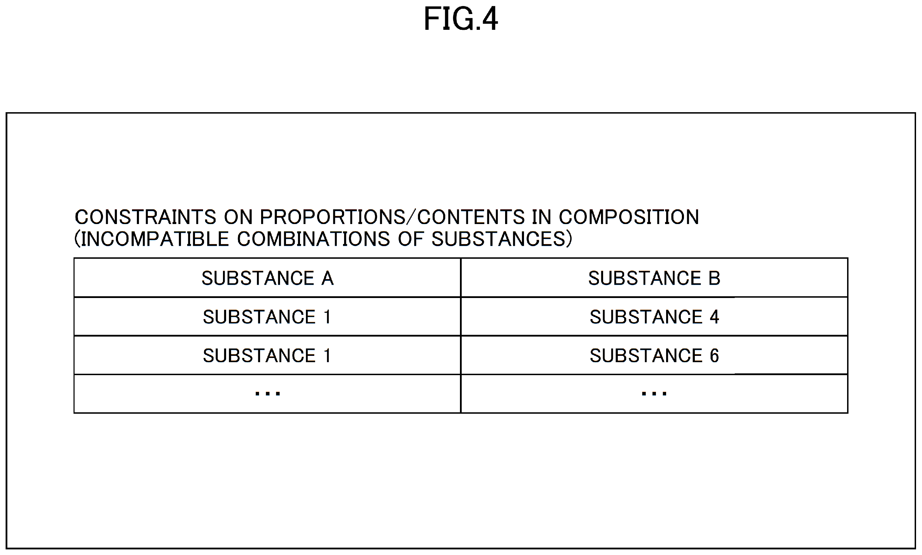

Furthermore, according to the present embodiment, the constraints on a composition shown in FIG. 4 are used. FIG. 4 is a diagram for explaining examples of constraints on a composition. To be more specific, FIG. 4 shows examples of conditions regarding combination of substances included in the substance groups shown in FIG. 3. Assuming a situation in which there are substances that are mutually incompatible, combinations of substances to be avoided are, for example, the ones shown in FIG. 4. As in FIG. 4, combinations of substances to be avoided may be identified within the same substance group (for example, the combination of substance 1 and substance 4 in FIG. 3) or identified across different substance groups (for example, the combination of substance 1 and substance 6 in FIG. 3).

The present embodiment is directed to finding an optimal composition of a composite material that satisfies the constraints of FIG. 3 and FIG. 4 and that optimizes the composite material's properties.

### <Functional Configuration>

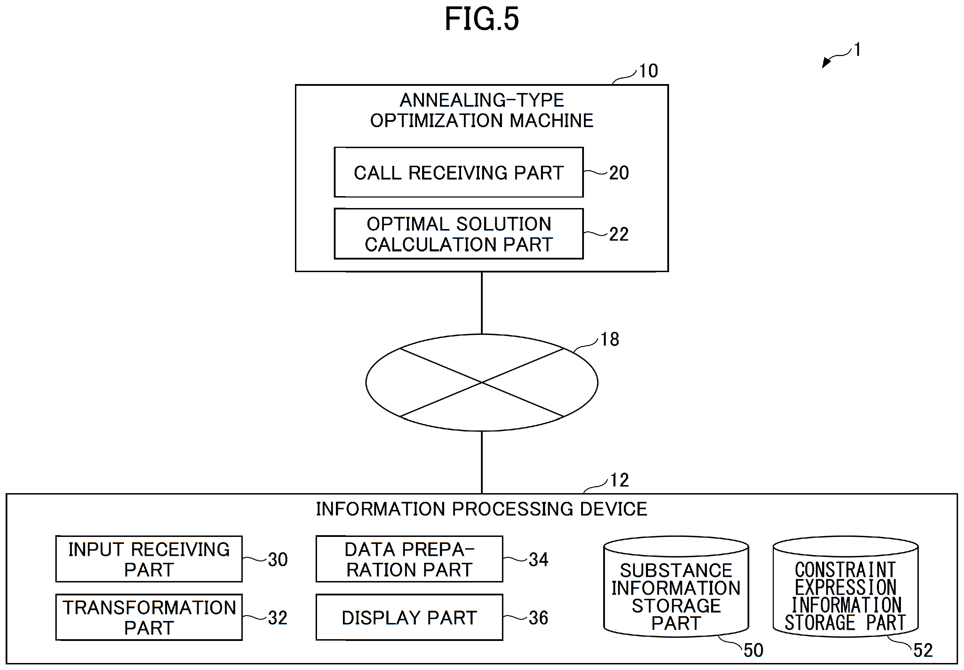

The architecture of the information processing system 1 according to the present embodiment will be described. FIG. 5 is a diagram showing an example architecture of an information processing system according to the present embodiment. Note that parts that are not necessary for explaining the present embodiment are omitted in the configurational diagram of FIG. 5.

The annealing-type optimization machine 10 shown in FIG. 5 includes at least a call receiving part 20 and an optimal solution calculation part 22. The information processing device 12 includes at least an input receiving part 30, a transformation part 32, a data preparation part 34, a display part 36, a substance information storage part 50, and a constraint expression information storage part 52.

The input receiving part 30 is an input interface where user operations are entered. The input receiving part 30 receives, as input from the user, information that is needed to allow the annealing-type optimization machine 10 to solve a combination optimization problem. For example, an objective function that formulates the target composite material's properties according to the proportions or contents of substances is input from the input receiving part 30.

The objective function formulates the composite material's properties according to the proportions or contents of substances contained therein, and is configured such that the smaller the value of the objective function, the nearer the user is to the desired properties. Examples of properties the user desires include, for example, high performance, low cost, etc. For example, the input receiving part 30 receives, as input, constraints on a composition, such as those shown in FIG. 3 and FIG. 4.

The transformation part 32 transforms the constraints on a composition into constraint expressions. A constraint expression is an Ising mathematical expression in which a constraint on proportions or contents in composition is formulated. A constraint expression may be arranged, for example, to yield 0 when the represented constraint is satisfied and yield a larger value when the constraint is not satisfied.

For example, a constraint expression may be formulated, for each substance group that constitutes at least part of a composite material, such that it yields a larger value when at least one of: a condition for the number of substances to be mixed from the substance group in the composite material; and a condition regarding the quantity of substances to be mixed in the composite material, is not satisfied.

The following mathematical expressions 6 and 7 represent, respectively, a constraint as an equation and a constraint as an inequality, and both can be represented as an Ising mathematical expression: [Math 8] $\begin{matrix}{\sum }_{i} {c}_{i} {x}_{i} = k & \left(6\right) \\ {\sum }_{i} {d}_{i} {x}_{i} \leq l & \left(7\right)\end{matrix} ,$ where cᵢ, k and dᵢ, l are real numbers, and xᵢ is 0 or 1.

The data preparation part 34 prepares an Ising model E, such as a mathematical expression 8 shown below, that allows the annealing-type optimization machine 10 to find an optimal solution, from the objective function and the constraint expressions:$E = {E}_{1} + {E}_{2}$ (8),
where E₁ is the objective function written in Ising form, and E₂ is the constraint expressions written in Ising form. E₂ assumes a number that matches the number of constraints on a composition, and is included in the Ising model E as one or more terms (constraint terms).

Furthermore, since E₁ and E₂ are Ising expressions, they can be expressed in QUBO form as well, as shown with the following mathematical expression 9:
[Math 9] $E = {\sum }_{i = 1}^{N} {\sum }_{j = i + 1}^{N} {J}_{ij} {x}_{i} {x}_{j} + {\sum }_{i = 1}^{N} {h}_{i} {x}_{i}$ (9), where xᵢ is 0 or 1 and Jᵢⱼ and hᵢ are real numbers.

From mathematical expression 8 given above, the data preparation part 34 prepares input information in a data format so that the annealing-type optimization machine 10 can use it, and sends the input information to the annealing-type optimization machine 10. The display part 36 displays the optimal solution received from the annealing-type optimization machine 10 on the display device 502 to have the user check it. The optimal solution displayed on the display device 502 may, for example, show information about the composition of the composite material in a user-friendly manner.

The substance information storage part 50 stores the properties (performance, cost, etc.) of substances included in the substance groups. For example, the level of a property that a composite material made up of substances has as a whole can be determined by multiplying the property of each substance by the substance's proportion or content ratio and finding the sum of the respective products.

The call receiving part 20 of the annealing-type optimization machine 10 receives a call from the information processing device 12. The call receiving part 20 also receives, from the information processing device 12, input information for the annealing-type optimization machine 10, prepared based on mathematical expression 8 given above.

Based on the input information the call receiving part 20 receives, the optimal solution calculation part 22 obtains {xᵢ} that minimizes the Ising model E. Obtaining {xᵢ} that minimizes the Ising model E is equivalent to finding a composition of the composite material in which the constraints on its composition, represented by E₂, are satisfied, and at which the objective function represented by E₁ is minimized.

Thus, where constraints on a composite material's composition are formulated into constraint expressions, the optimal solution calculation part 22 can determine a composition, as an optimal solution, in which the composite material has optimal properties. The call receiving part 20 sends the optimal solution determined by the optimal solution calculation part 22 to the information processing device 12.

Note that the configurational diagram of FIG. 5 is one example. Various architectures may be available for the information processing system 1 according to the present embodiment. In addition, although an example has been described above with the present embodiment in which the input receiving part 30 receives constraints on a composition as input, the input receiving part 30 may receive formulated constraints as input.

### <Bit Representations Related to Substances>

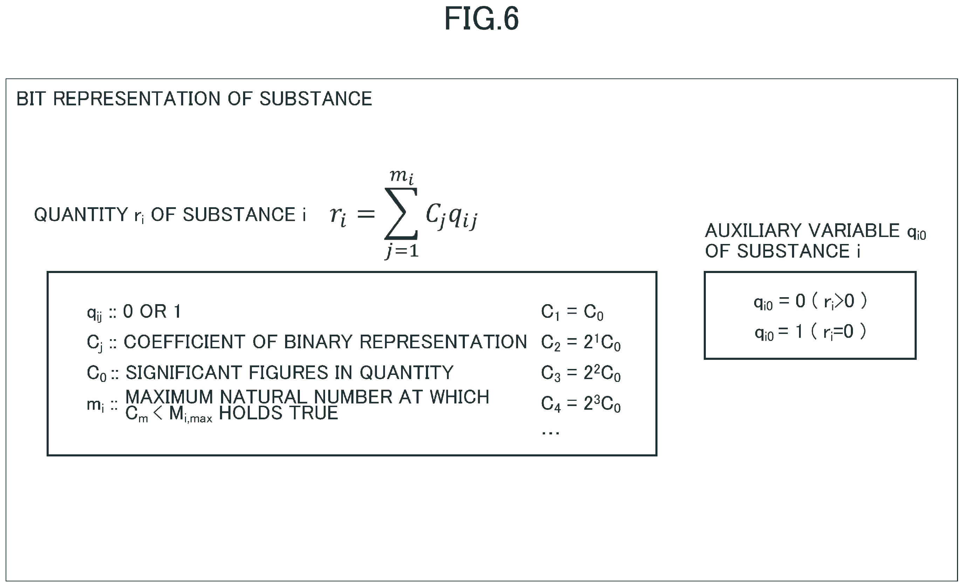

The bit representations in the following description represent the quantity of individual substances and are needed when expressing constraints on a composition using Ising models. For example, the bit representations shown in FIG. 6 may be used with respect to a substance.

FIG. 6 is a diagram for explaining examples of bit representations related to a substance. For example, given a substance i, the following mathematical expression 10 provides a bit representation of substance i's quantity rᵢ, and, furthermore, provides a bit representation, namely substance i's auxiliary variable qᵢ₀, that indicates whether substance i is contained in the composite material:
[Math 10] ${r}_{i} = {\sum }_{j = 1}^{{m}_{i}} {C}_{j} {q}_{ij}$ (10), where:
qᵢⱼ is a binary number, that is, 0 or 1, that indicates the proportion or the content of substance i;
Cⱼ is a coefficient for a bit qᵢⱼ in the binary representation of the composition of substance i;
C₀ is the significant figures in the quantity of the substance mixed;
mᵢ is the maximum natural number at which Cₘ < M_{i,MAX} holds true;
M_{i,MAX} is the maximum level for the quantity rᵢ of substance i; and
M_{i,MIN} is the minimum level for the quantity rᵢ of substance i.

If the quantity rᵢ of substance i is not 0 (meaning that substance i is contained in the composite material), the auxiliary variable qᵢ₀ is 0. If the quantity rᵢ of substance i is 0 (meaning that substance i is not contained in the composite material), the auxiliary variable qᵢ₀ is 1.

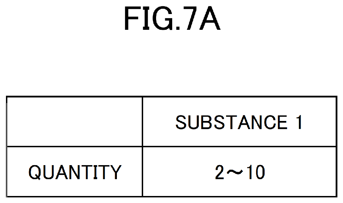

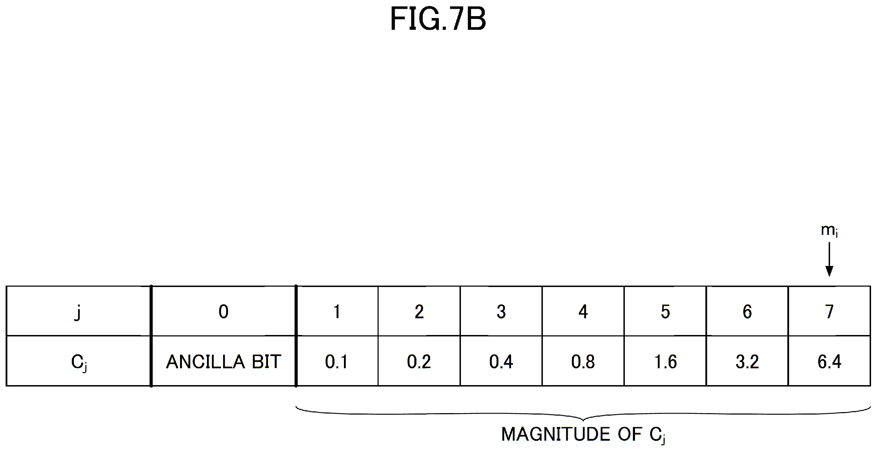

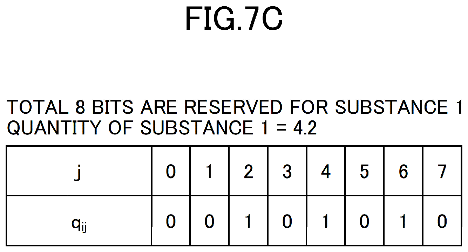

For example, assuming that there is a substance 1; its significant figure is one decimal place and its quantity ranges from 2 to 10. This substance 1 can be represented using the bit representations shown in FIG. 7A to FIG. 7C. FIG. 7A to FIG. 7C are diagrams for explaining bit representations related to substance 1 with specific examples.

The maximum quantity M_{i,MAX} of substance 1 is 10. Therefore, mᵢ, which is the maximum natural number at which Cₘ < M_{i,MAX} holds true, is 7. The number of bits that need to be reserved for substance 1, including an ancilla bit for representing an auxiliary variable nᵢ₀, is 8 bits in total.

For example, if the quantity of substance 1 is 4.2, the value of the ancilla bit is 0 because substance 1 is contained in the composite material. Among the bit values (shown as "j," which ranges from 1 to 7) for representing substance 1's quantity "4.2," the bit "j = 2" representing the quantity "0.2," the bit "j = 4" representing the quantity "0.8," and the bit "j = 6" representing the quantity "3.2" are all 1.

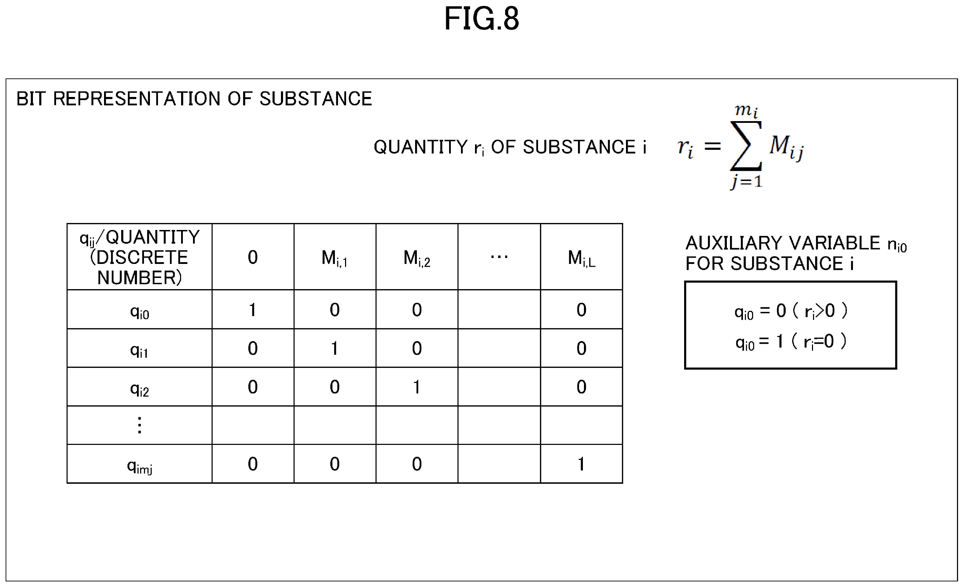

Furthermore, the bit representations related to a substance shown in FIG. 8 may be applicable as well. FIG. 8 is a diagram for explaining examples of bit representations related to a substance. The following mathematical expression 11 provides a bit representation of substance i's quantity rᵢ, and, furthermore, provides a bit representation that indicates whether substance i is contained in the composite material, namely substance i's auxiliary variable nᵢ₀:
[Math 11] ${r}_{i} = {\sum }_{j = 1}^{{m}_{i}} {M}_{ij}$ (11).

If the quantity of substance i is represented by a discrete number such as one among "M_{i,1}, M_{i,2}, ..., M_{i,L}," the bit representing M_{i,L} provides a bit representation of the quantity rᵢ of substance i. The auxiliary variable nᵢ₀ of substance i provides a bit representation that indicates whether or not substance i is contained in the composite material.

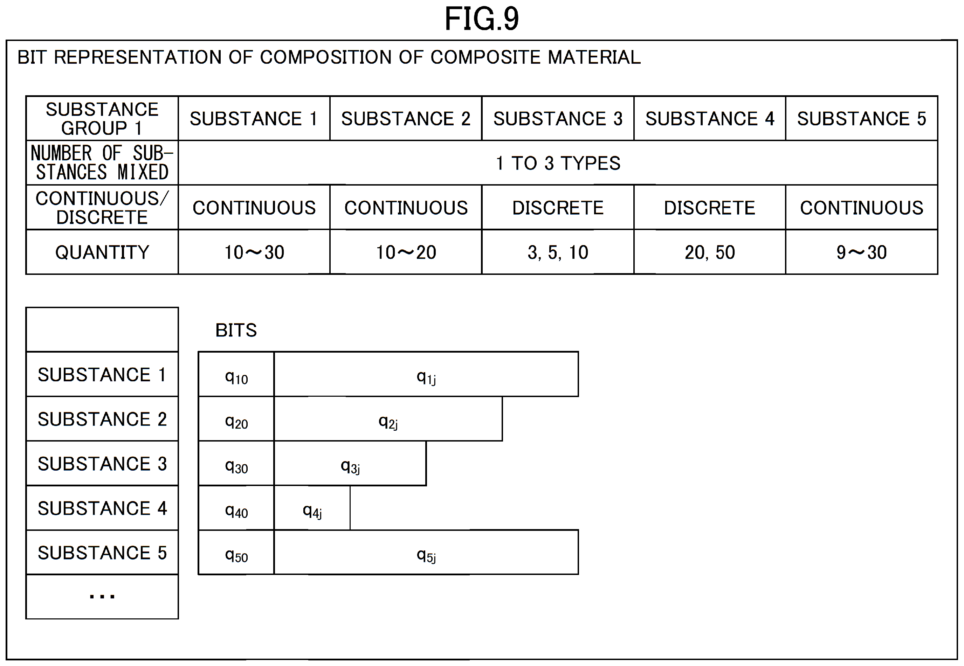

Using the bit representations described above to represent the quantity of each constituent substance, the composition of the target composite material can be represented using the bit representations shown in FIG. 9. FIG. 9 is a diagram for explaining examples of bit representations related to a composite material's composition.

FIG. 9 shows an example in which substance group 1, which constitutes at least part of a composite material, contains five types of substances, namely substance 1 to substance 5. Substance 1, substance 2, and substance 5 are examples of substances, the quantity of which is represented by continuous numbers. Substance 3 and substance 4 are examples of substances, the quantity of which is represented by discrete numbers.

As explained earlier, the number of bits that need to be reserved for substance 1 to substance 5 is determined by: whether the quantity of each substance mixed in the target composite material is set with a continuous number or a discrete number; and the quantity of each substance. q₁ⱼ to q₅ⱼ are bit representations of the respective quantities of substance 1 to substance 5. For example, if the minimum unit of quantity is 0.1 and the maximum quantity of substance 1 is 30, the quantity of substance 1 can be represented by bit representations q₁₁ to q₁₉. Similarly, the quantity of substance 3 can be given by three discrete numbers "3," "5," and "10," and can be represented by bit representations q₃₁ to q₃₃.

When the annealing-type optimization machine 10 is used, the bit representations may be transformed into the one-dimensional vector representation {xᵢ} of mathematical expression 9. By connecting all the bit representations related to respective substances from all of the substance groups constituting the target composite material, the composite material's composition can be expressed using a one-dimensional vector representation {xᵢ}, as shown with mathematical expression 9.

### <Constraints>

When a composite material is made up of multiple substance groups in a certain composition and a combination optimization problem needs to be solved to determine a composition, that is, a combination of proportions or contents of individual substances, in which the composite material has optimal properties, the following constraints may be applied:

### <<Constraint Set 1>>

In this embodiment, constraints based on the following mathematical expressions 12 and 13 are applied per substance i:
[Math 12] $\left\{\begin{matrix}\left({m}_{i}+1\right) {q}_{i 0} + {\sum }_{j = 1}^{{m}_{i}} {q}_{ij} \leq {m}_{i} + 1 & \left(12\right) \\ \left({m}_{i}+1\right) {q}_{i 0} + {\sum }_{j = 1}^{{m}_{i}} {q}_{ij} \geq 1 & \left(13\right)\end{matrix}\right. .$

If the quantity of substance i is represented by a continuous number, then, mᵢ included in mathematical expressions 12 and 13 is the maximum natural number at which Cₘ < M_{i, Max} holds true. If the quantity of substance i is represented by a discrete number, then, mᵢ included in mathematical expressions 12 and 13 is the number of discrete numbers that each represent the quantity rᵢ of substance i. mᵢ+1 therefore represents the number of bits reserved for substance i, plus an ancilla bit representing the auxiliary variable qᵢ₀.

The constraint represented by the inequality of mathematical expression 12 determines that the second term on the left-hand side is 0 when the auxiliary variable gᵢ₀ on the left-hand side is 1 and determines that the second term on the left-hand side ranges from 1 to mᵢ when the auxiliary variable qᵢ₀ on the left-hand side is 0.

The constraint represented by the inequality of mathematical expression 13 determines that the second term on the left-hand side is not 0 when the auxiliary variable qᵢ₀ on the left-hand side is 0. The constraints represented by mathematical expressions 12 and 13, expressed as inequalities, ensure that: substance i's auxiliary variable qᵢ₀ is 0 when rᵢ > 0 holds true; and, substance i's auxiliary variable qᵢ₀ is 1 when rᵢ = 0 holds true.

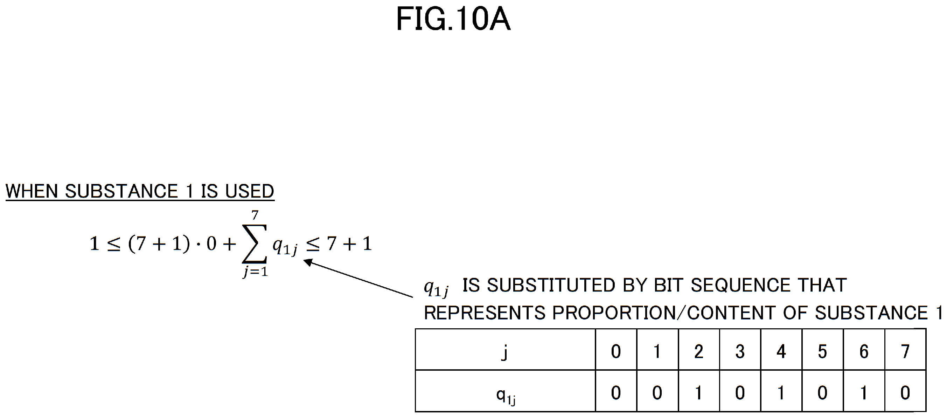

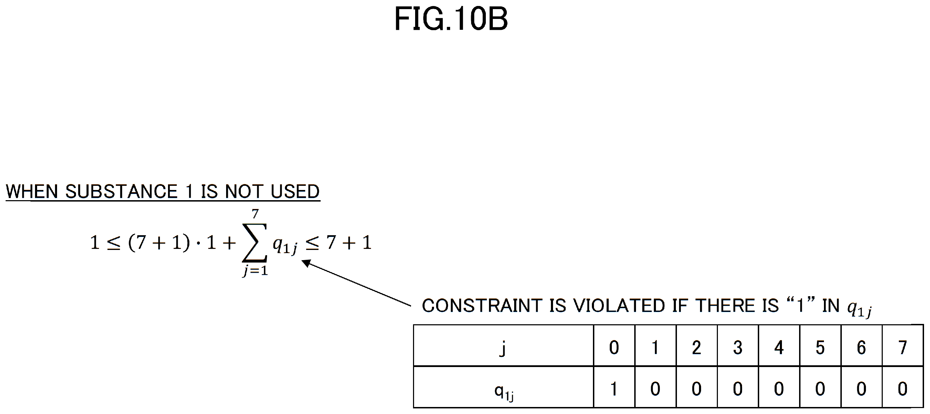

FIG. 10A and FIG. 10B are diagrams for explaining constraints based on mathematical expressions 12 and 13 with examples. FIG. 10A and FIG. 10B show examples of calculating mathematical expressions 12 and 13 for substance 1. The quantity of substance 1 ranges from 2 to 10, and mᵢ is 7.

When substance 1 is used, as shown in the table of FIG. 10A, if a bit "1" is included in the bit sequence representing the proportion or the content of substance 1, the corresponding constraint is satisfied. On the other hand, if a bit "1" is not included in the bit sequence representing the proportion or the content of substance 1, the corresponding constraint is violated.

When substance 1 is not used, as shown in the table of FIG. 10B, if a bit "1" is not included in the bit sequence representing the proportion or the content of substance 1, the corresponding constraint is satisfied. On the other hand, if a bit "1" is included in the bit sequence representing the proportion or the content of substance 1, the corresponding constraint is violated.

The constraints represented by mathematical expressions 12 and 13 are thus applied per substance i. For example, if there are 20 substances i, since 20 × 2 = 40, 40 constraints need to be applied in total. A constraint set 1 is thus comprised of constraints that relate to the auxiliary variable qᵢ₀.

### <<Constraint Set 2>>

In this embodiment, assuming that certain substances are mutually incompatible and not to be combined together, constraints based on the following mathematical expressions 14 and mathematical expression 15 are applied to each combination to be avoided. An example will be described below in which a substance A and a substance B make an incompatible combination. The following mathematical expressions 14 and 15 represent constraints to the effect that substance B is not used when substance A is used. [Math 13] $\left\{\begin{matrix}\left({m}_{B}+1\right) \left(1-{q}_{A 0}\right) + {\sum }_{j = 1}^{{m}_{i}} {q}_{Bj} \leq {m}_{B} + 1 & \left(14\right) \\ \left({m}_{B}+1\right) \left(1-{q}_{A 0}\right) + {\sum }_{j = 1}^{{m}_{i}} {q}_{Bj} \geq 1 - {q}_{B 0} & \left(15\right)\end{matrix}\right. .$

If the quantity of substance B is represented by a continuous number, then, m_{b} included in mathematical expressions 14 and 15 is the maximum natural number at which Cₘ < M_{i, Max} holds true. On the other hand, if the quantity of substance B is represented by a discrete number, then, m_{B} included in mathematical expressions 14 and 15 is the number of discrete numbers that each represent the quantity rᵢ of substances B. m_{B}+1 therefore represents the number of bits reserved for substance B, plus an ancilla bit representing an auxiliary variable q_{B0}.

The constraint represented by the inequality of mathematical expression 14 determines that the second term on the left-hand side ranges from 0 to m_{B} when the auxiliary variable q_{A0} on the left-hand side is 1, and determines that the second term on the left-hand side is 0 when the auxiliary variable q_{A0} on the left-hand side is 0.

The constraint represented by the inequality of mathematical expression 15 allows the second term on the left-hand side to be 0 when the auxiliary variable q_{A0} on the left-hand side is 1. The constraints represented by mathematical expressions 14 and 15, expressed as inequalities, ensure that: substance i's auxiliary variable qᵢ₀ is 0 when rᵢ > 0 holds true; and substance i's auxiliary variable qᵢ₀ is 1 when rᵢ = 0 holds true.

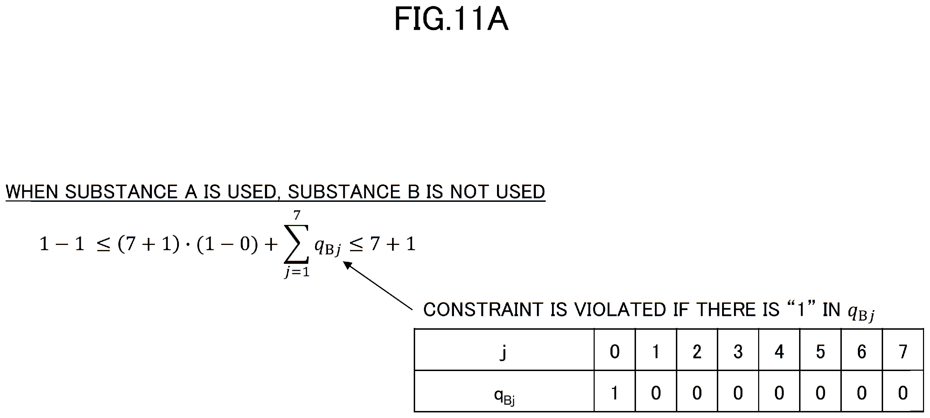

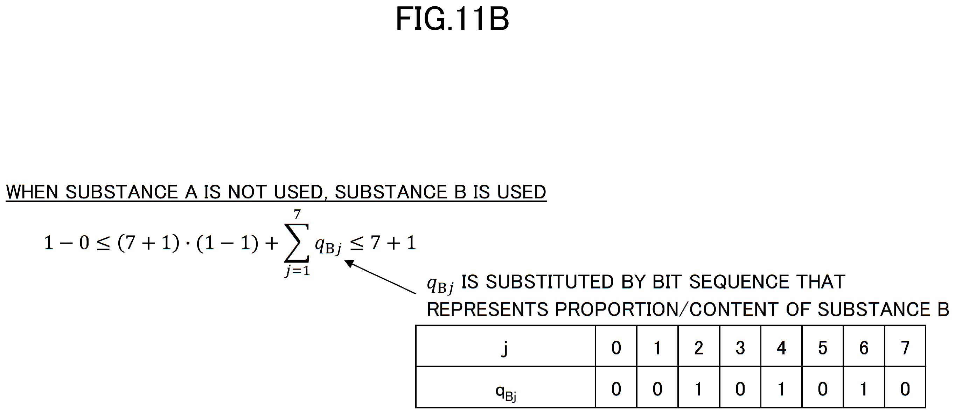

FIG. 11A and FIG. 11B are diagrams for explaining the constraint represented by mathematical expressions 14 and 15 with examples. FIG. 11A shows an example of calculating mathematical expressions 14 and 15 for substance B in the event substance A is used. The quantity of substance B ranges from 2 to 10, and mᵢ is 7.

Referring to the table of FIG. 11A, the constraint "when substance A is used, substance B is not used" is satisfied if a bit "1" is not included in the bit sequence representing the proportion or the content of substance B. On the other hand, if a bit "1" is included in the bit sequence representing the proportion or the content of substance B, the constraint "when substance A is used, substance B is not used" is violated.

Likewise, as shown in the table of FIG. 11B, the constraint "when substance A is not used, substance B is used" is satisfied if a bit "1" is included in the bit sequence representing the proportion or the content of substance B. On the other hand, if a bit "1" is not included in the bit sequence representing the proportion or the content of substance B, the constraint "when substance A is not used, substance B is used" is violated.

The constraints represented by mathematical expressions 14 and 15 are applied to each incompatible combination of substances. To make the opposite hold true, constraints based on the following mathematical expression 16 and mathematical expression 17 are applied to substance A. Mathematical expressions 16 and 17 represent constraints to the effect that substance A is not used when substance B is used: [Math 14] $\left\{\begin{matrix}\left({m}_{A}+1\right) \left(1-{q}_{B 0}\right) + {\sum }_{j = 1}^{{m}_{i}} {q}_{Aj} \leq {m}_{A} + 1 & \left(16\right) \\ \left({m}_{A}+1\right) \left(1-{q}_{B 0}\right) + {\sum }_{j = 1}^{{m}_{i}} {q}_{Aj} \geq 1 - {q}_{A 0} & \left(17\right)\end{matrix}\right. .$

If the quantity of substance A is represented by a continuous number, then, mₐ in mathematical expressions 16 and 17 is the maximum natural number at which Cₘ < M_{i, MAX} holds true. If the quantity of substance A is represented by a discrete number, then, m_{A} in mathematical expressions 16 and 17 is the number of discrete numbers that each represent the quantity rᵢ of substance A. m_{A}+1 therefore represents the number of bits reserved for substance A, plus an ancilla bit representing the auxiliary variable q_{A0}.

The constraint represented by the inequality of mathematical expression 16 determines that the second term on the left-hand side ranges from 0 to m_{A} when the auxiliary variable q_{B0} on the left-hand side is 1, and that the second term on the left-hand side is 0 when the auxiliary variable q_{B0} on the left-hand side is 0.

The constraint represented by the inequality of mathematical expression 17 allows the second term on the left-hand side to range from 0 to m_{B} when the auxiliary variable q_{B0} on the left-hand side is 1. The constraints represented by mathematical expressions 16 and 17, expressed as inequalities, ensure that: substance i's auxiliary variable qᵢ₀ is 0 when rᵢ > 0 holds true; and substance i's auxiliary variable qᵢ₀ is 1 when rᵢ = 0 holds true.

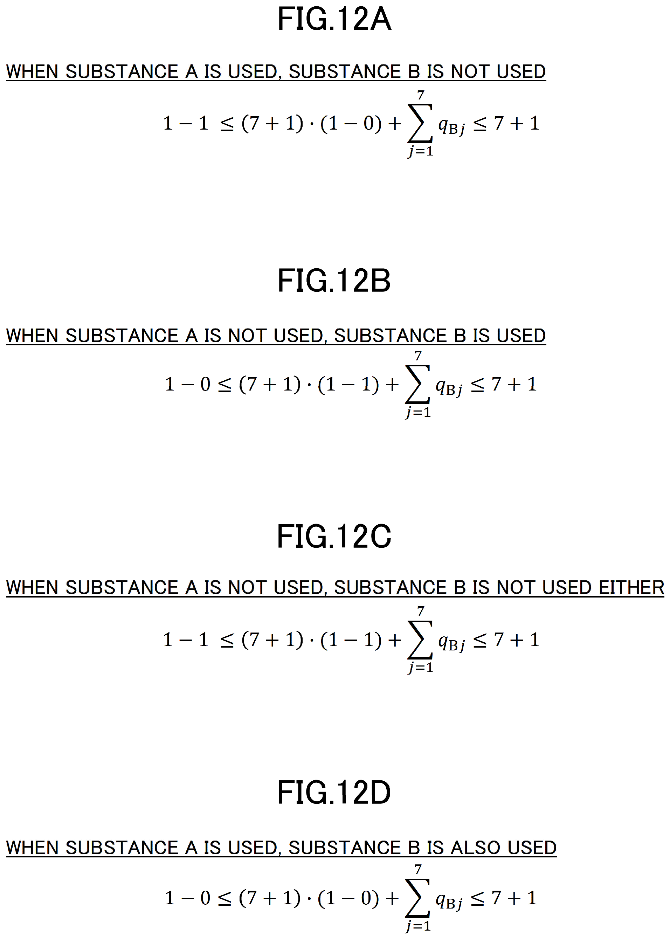

A specific example of the present embodiment will be further described. FIG. 12A to FIG. 12D are diagrams for explaining example usages of the constraints represented by mathematical expressions 14 and 15. In the examples of FIG. 12A to FIG. 12D, m_{B} of substance B is 7.

In FIG. 12A, the constraint "when substance A is used, substance B is not used" is satisfied when: the auxiliary variable q_{A0} is 0; the auxiliary variable q_{B0} is 1; and a bit "1" is not included in the bit sequence representing the proportion or the content of substance B.

In FIG. 12B, the constraint "when substance A is not used, substance B is used" is satisfied when: the auxiliary variable q_{A0} is 1; the auxiliary variable q_{B0} is 0; and a bit "1" is included in the bit sequence representing the proportion or the content of substance B.

In FIG. 12C, the constraint "when substance A is not used, substance B is not used either" is satisfied when: the auxiliary variable q_{A0} is 1; the auxiliary variable q_{B0} is 1; and a bit "1" is not included in the bit sequence representing the proportion or the content of substance B.

In FIG. 12D, the constraint "when substance A is used, substance B is also used" is not satisfied but violated when: the auxiliary variable q_{A0} is 0; the auxiliary variable q_{B0} is 0; and a bit "1" is included in the bit sequence representing the proportion or the content of substance B.

Examples of imposing constraints on substance B have been described with reference to FIG. 12A to FIG. 12D. To make the opposite of these hold true, examples of imposing similar constraints on substance A will be described below.

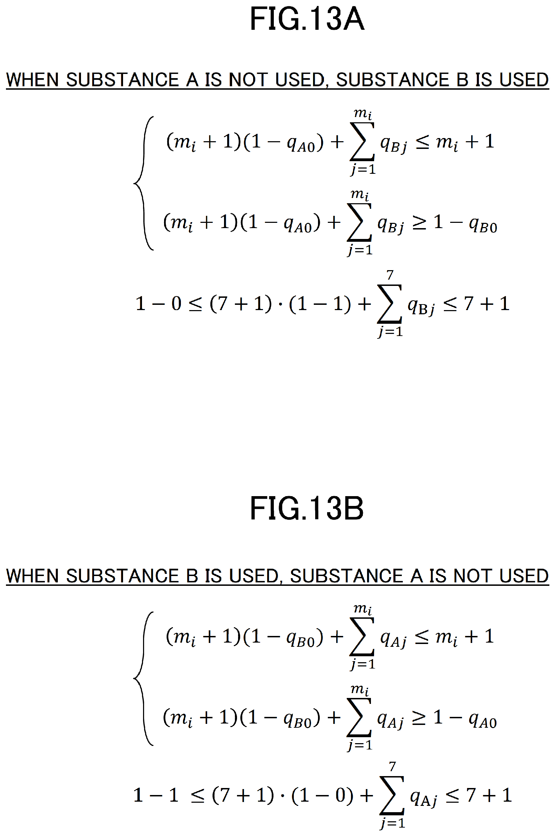

FIG. 13A and FIG. 13B are diagrams for explaining example usages of the constraints represented by mathematical expressions 14, 15, 16, and 17. In the examples shown in FIG. 13A and FIG. 13B, m_{A} and m_{B} are 7.

In FIG. 13A, the constraint "when substance A is not used, substance B is used" is satisfied when: the auxiliary variable q_{A0} is 0; the auxiliary variable q_{B0} is 1; and a bit "1" is not included in the bit sequence representing the proportion or the content of substance B.

In FIG. 13B, the constraint "when substance B is used, substance A is not used" is satisfied when: the auxiliary variable q_{A0} is 1; the auxiliary variable q_{B0} is 0; and a bit "1" is not included in the bit sequence representing the proportion or the content of substance A.

Thus, the constraints represented by mathematical expressions 16 and 17, which are the opposite of the constraints represented by mathematical expressions 14 and 15, also hold true.

According to the present embodiment, for example, when there are substances that are mutually incompatible and not to be combined together, it is possible to apply constraints per combination to be avoided, thereby preventing an incompatible combination of substances from being selected as an optimal combination. For example, if certain substances do not mix well due to poor compatibility (poor miscibility, for example) and what substances are not to be combined together are known in advance, then, constraints may be applied as described above, so that, for example, unnecessary searches can be avoided.

### <<Constraint Set 3>>

In this embodiment, if there are substances that are mutually incompatible and not to be combined together, the constraints represented by mathematical expressions 12 and 13 described above are applied per substance i, and, in addition, constraints based on the following mathematical expression 18 are applied to substances i to be constrained:
[Math 15] ${\sum }_{i = 1}^{N} {q}_{i 0} \geq N - 1$ (18)

N included in mathematical expression 18 is the number of substances i subject to constraints based on mathematical expression 18. The left-hand side is the number of unused substances i among substances i subject to constraints. The inequality of mathematical expression 18 represents a constraint for determining whether the number of unused substances i is greater than or equal to N-1.

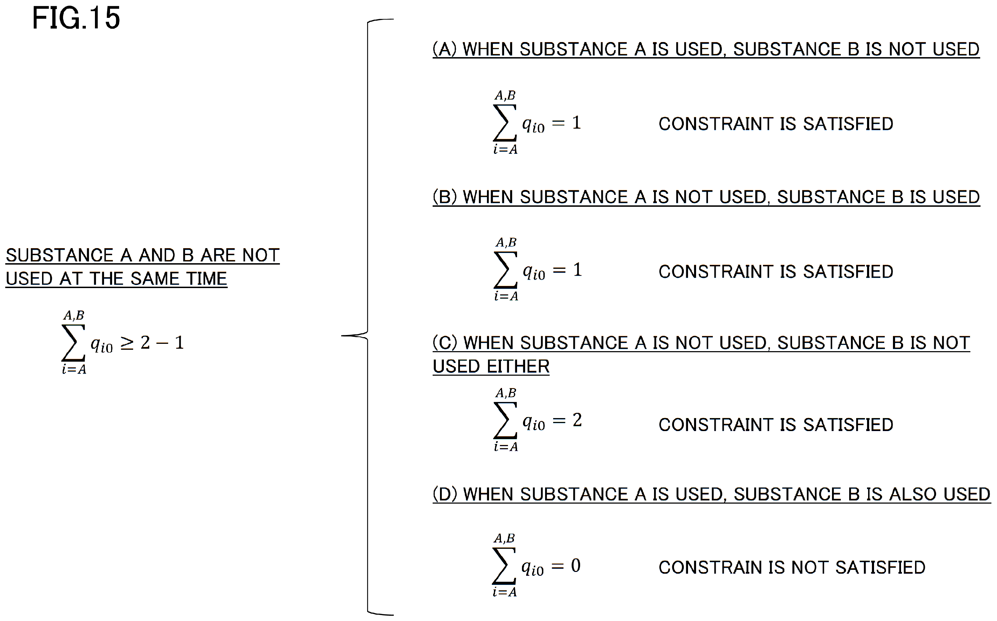

An example will be described here in which substance A and substance B make an incompatible combination and therefore are not used at the same time. FIG. 15 is a diagram for explaining example usages of constraints based on mathematical expression 18. FIG. 15 illustrates examples in which two substances i are subject to constraints based on mathematical expression 18.

In (A) in FIG. 15, the constraint "when substance A is used, substance B is not used" is satisfied when: the auxiliary variable q_{A0} is 0; the auxiliary variable q_{B0} is 1; and the left-hand side is 1.

In (B) in FIG. 15, the constraint "when substance A is not used, substance B is used" is satisfied when: the auxiliary variable q_{A0} is 1; the auxiliary variable q_{B0} is 0; and the left-hand side is 1.

In (C) in FIG. 15, the constraint "when substance A is not used, substance B is not used either" is satisfied when: the auxiliary variable q_{A0} is 1; the auxiliary variable q_{B0} is 1; and the left-hand side is 2.

In (D) in FIG. 15, the constraint "when substance A is used, substance B is also used" based on mathematical expression 18 is not satisfied but violated when: the auxiliary variable q_{A0} is 0; the auxiliary variable q_{B0} is 0; and the left-hand side is 0.

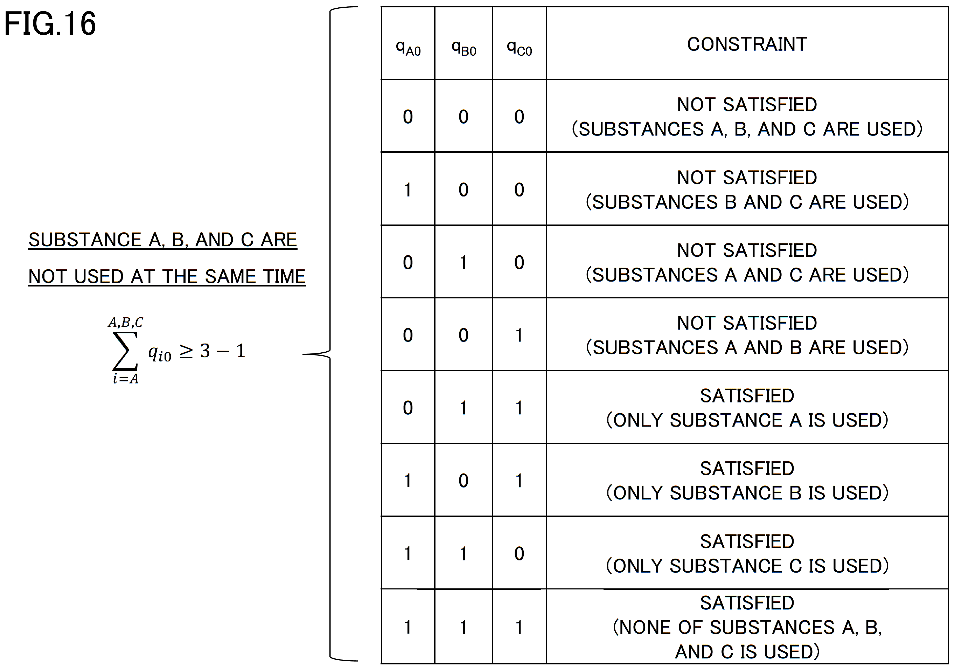

Next, an example in which substance A, substance B, and substance C make incompatible combinations and therefore are not used at the same time will be described. FIG. 16 is a diagram for explaining example usages of constraints based on mathematical expression 18 given earlier. In addition, FIG. 16 shows an example in which 3 substances i are subject to constraints based on mathematical expression 18.

Assuming that substance A, substance B, and substance C are either "used" or "not used," these statuses may be combined together in 8 patterns as shown in the table of FIG. 16. The auxiliary variable q_{A0} is 0 when substance A is used and 1 when substance A is not used. The auxiliary variable q_{B0} is 0 when substance B is used and 1 when substance B is not used. The auxiliary variable q_{C0} is 0 when substance C is used and 1 when substance C is not used.

The constraint "none of substance A, B, and C is used" is satisfied when: the auxiliary variable q_{A0} is 1; the auxiliary variable q_{B0} is 1; the auxiliary variable q_{C0} is 1; and the left-hand side is 3.

The constraint "only substance A is used" is satisfied when: the auxiliary variable q_{A0} is 0; the auxiliary variable q_{B0} is 1; the auxiliary variable q_{C0} is 1; and the left-hand side is 2.

The constraint "only substance B is used" is satisfied when: the auxiliary variable q_{A0} is 1; the auxiliary variable q_{B0} is 0; the auxiliary variable q_{C0} is 1; and the left-hand side is 2.

The constraint "only substance C is used" is satisfied when: the auxiliary variable q_{A0} is 1; the auxiliary variable q_{B0} is 1; the auxiliary variable q_{C0} is 0; and the left-hand side is 2.

The constraint "substance B and C are used," represented by mathematical expression 18, is not satisfied but violated when: the auxiliary variable q_{A0} is 1; the auxiliary variable q_{B0} is 0; the auxiliary variable q_{C0} is 0; and the left-hand side is 1.

The constraint "substance A and C are used," represented by mathematical expression 18, is not satisfied but violated when: the auxiliary variable q_{A0} is 0; the auxiliary variable q_{B0} is 1; the auxiliary variable q_{C0} is 0; and the left-hand side is 1.

The constant "substance A and B are used," based on mathematical expression 18, is not satisfied but violated when: the auxiliary variable q_{A0} is 0; the auxiliary variable q_{B0} is 0; the auxiliary variable q_{C0} is 1; and the left-hand side is 1.

The constant "substance A, B, and C are used," based on mathematical expression 18, is not satisfied but violated when: the auxiliary variable q_{A0} is 0; the auxiliary variable q_{B0} is 0; the auxiliary variable q_{C0} is 0; and the left-hand side is 0.

According to the present embodiment, for example, when certain substances are mutually incompatible and not to be combined together, constraints can be applied to prevent an incompatible combination of substances from being selected as an optimal combination. For example, if certain substances do not mix well due to poor compatibility (poor miscibility, for example) and what substances are not to be combined together are known in advance, then, constraints may be applied as described above, so that, for example, unnecessary searches can be avoided.

### <Processing>

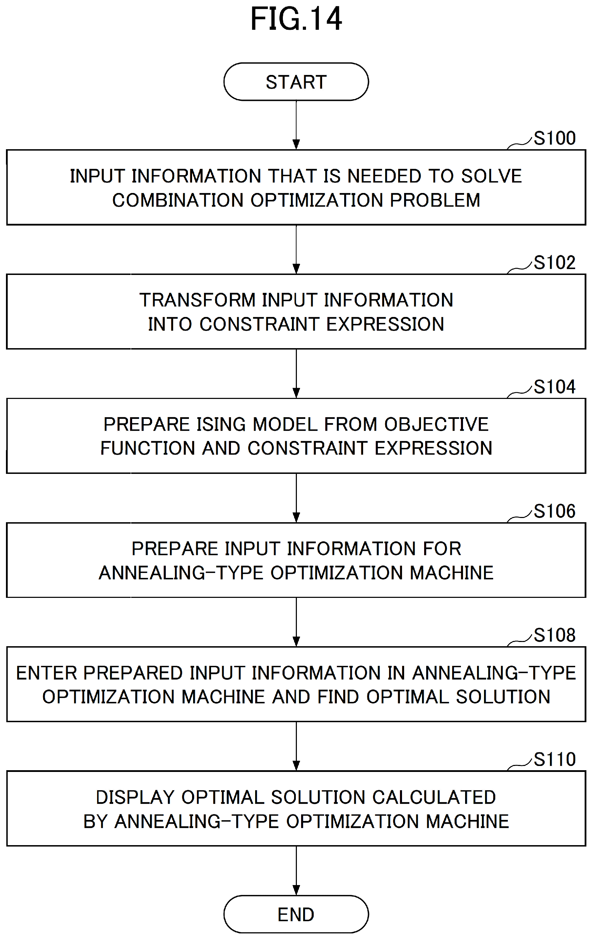

FIG. 14 is a flowchart showing examples of processing steps executed by the information processing system of the present embodiment.

In step S100, the information processing device 12 receives, as input from the user, information that is needed to allow the annealing-type optimization machine 10 to solve a combination optimization problem. For example, the information processing device 12 receives, as input, an objective function that formulates an object material's properties according to the composition of substances in the composite material. The information processing device 12 also receives, as input, a constraint on the composition of the composite material.

In step S102, the information processing device 12 transforms the constraint on the proportions or contents in the composition of the composite material received in step S100 into a constraint expression. The information processing device 12 transforms the constraint on the proportions or contents in the composition of the composite material into a constraint expression based on information about constraint expressions defined with respect to substances i and stored in the constraint expression information storage part 52. Note that the user may perform the process of transforming the constraint on the proportions or contents in the composition of the composite material into a constraint expression.

In step S104, the information processing device 12 prepares an Ising model to be solved by the annealing-type optimization machine 10, from the objective function and the constraint expression.

In step S106, the information processing device 12 prepares input information in a data format so that the annealing-type optimization machine 10 can use it, based on the Ising model prepared in step S104, and sends the input information to the annealing-type optimization machine 10. The input information in a data format that the annealing-type optimization machine 10 supports includes components that make up the objective function and the constraint expression. The input information for the annealing-type optimization machine 10 is, for example, an electronic file to be sent to the annealing-type optimization machine 10.

In step S108, the information processing device 12 sends the input information for the annealing-type optimization machine 10 to the annealing-type optimization machine 10. Based on the input information received, the annealing-type optimization machine 10 determines an optimal solution (composition of substances in which the composite material's properties are optimized) from among solutions that satisfy the constraint (constraint on the composition of the composite material).

In step S110, the annealing-type optimization machine 10 sends information that represents the optimal solution determined, to the information processing device 12. The information processing device 12 receives information (bit information) about the optimal solution from the annealing-type optimization machine 10, transforms it into user-friendly information such as a composition of substances in the composite material, and outputs this information. For example, the information processing device 12 may display the composition of (for example, the names of substances in) the composite material according to the optimal solution, with the quantity of each substance.

The composition of the composite material found as an optimal solution according to this embodiment can be used to control a device that produces a composite material by specifying the substances to be mixed and the quantity of each substance. A composite material preparing device such as an aluminum alloy manufacturing device may be one example. The present embodiment can also be used to search for proportions or contents in the composition of a semiconductor material as an example of a composite material. This semiconductor material is, for example, a resist material, an adhesive, a pressure-sensitive adhesive, or an encapsulant, and is a composite material made up of multiple resins, additives, and/or fillers.

As described above, the information processing system 1 according to the present embodiment can assist preparation of Ising models that will allow the annealing-type optimization machine 10 to solve an optimization problem for a composite material with constraints on proportions or contents in the composition of the composite material.

Although the present embodiment has been described above, it should be understood that various changes in form and technical specifics can be made without departing from the spirit and scope of the present disclosure set forth in the accompanying claims. The present invention has been described based on an embodiment, but the present invention is by no means limited to the embodiment described herein, and a variety of changes can be made within the scope of the present disclosure set forth in the accompanying claims. This application claims priority to Japanese Patent Application No. 2023-222639, filed with Japan Patent Office on December 28, 2023, and Japanese Patent Application No. 2024-033063, also filed with Japan Patent Office on March 5, 2024, the entire contents of which are incorporated herein by reference.

### Reference Signs List

- 1: information processing system
- 10: annealing-type optimization machine
- 12: information processing device
- 18: communication network
- 20: call receiving part
- 22: optimal solution calculation part
- 30: input receiving part
- 32: transformation part
- 34: data preparation part
- 36: display part
- 50: substance information storage part
- 52: constraint expression information storage part

## Claims

1. An information processing device configured to assist preparation of an Ising model for allowing an annealing-type optimization machine to solve an optimal solution search problem for a composite material with constraints on proportions or contents in a composition of the composite material, the information processing device comprising:
an input receiving part configured to receive as input:
an objective function that represents a property of the composite material according to the proportions or contents in the composition of the composite material; and
a constraint on the proportions or contents in the composition of the composite material; and
a data preparation part configured to prepare the Ising model that allows the annealing-type optimization machine to find an optimal solution for the proportions or contents in the composition of the composite material, the optimal solution satisfying a constraint expression that represents the constraint on the proportions or contents in the composition of the composite material and optimizing the property of the composite material,
wherein, when the composite material is a mixture of substances:
a quantity rᵢ of a substance i is indicated by a bit representation; and
whether or not the substance i is contained in the composite material is indicated by the bit representation,
wherein the constraint expression includes conditions that require that following mathematical expressions 1 and 2 be satisfied: [Math 1] $\left\{\begin{matrix}\left({m}_{B}+1\right) \left(1-{q}_{A 0}\right) + {\sum }_{j = 1}^{{m}_{i}} {q}_{Bj} \leq {m}_{B} + 1 & \left(1\right) \\ \left({m}_{B}+1\right) \left(1-{q}_{A 0}\right) + {\sum }_{j = 1}^{{m}_{i}} {q}_{Bj} \geq 1 - {q}_{B 0} & \left(2\right)\end{matrix}\right. ,$ where:
q_{A0} is an auxiliary variable that is 0 when a substance A is contained in the composite material and that is 1 when the substance A is not contained in the composite material;
q_{B0} is an auxiliary variable that is 0 when a substance B is contained in the composite material and that is 1 when the substance B is not contained in the composite material;
m_{B} is a number of bits required to represent a quantity r_{B} of the substance B to be mixed in the composite material; and
q_{Bj} is a number 0 or 1 of a binary representation of a proportion or content of the substance B, and
wherein the constraint expression is arranged such that, when a condition included in the constraint expression fails to satisfy at least one of the mathematical expression 1 or 2, a proportion or content represented by the failing condition is excluded from the optimal solution for the composition of the composite material.

2. The information processing device according to claim 1,
wherein the constraint expression further includes conditions that require that following mathematical expressions 3 and 4 be satisfied: [Math 2] $\left\{\begin{matrix}\left({m}_{A}+1\right) \left(1-{q}_{B 0}\right) + {\sum }_{j = 1}^{{m}_{i}} {q}_{Aj} \leq {m}_{A} + 1 & \left(3\right) \\ \left({m}_{A}+1\right) \left(1-{q}_{B 0}\right) + {\sum }_{j = 1}^{{m}_{i}} {q}_{Aj} \geq 1 - {q}_{A 0} & \left(4\right)\end{matrix}\right. ,$ where:
q_{A0} is the auxiliary variable that is 0 when the substance A is contained in the composite material and that is 1 when the substance A is not contained in the composite material;
q_{B0} is the auxiliary variable that is 0 when the substance B is contained in the composite material and that is 1 when the substance B is not contained in the composite material;
m_{A} is a number of bits required to represent a quantity r_{A} of the substance A to be mixed in the composite material; and
q_{Aj} is a number 0 or 1 of a binary representation of a proportion or content of the substance A in the composite material, and
wherein the constraint expression is arranged such that, when a condition included in the constraint expression fails to satisfy at least one of the mathematical expression 3 or 4, a proportion or content represented by the failing condition is excluded from the optimal solution for the composition of the composite material.

3. The information processing device according to claim 1 or 2, wherein, when a condition regarding a quantity of a substance to be mixed in the composite material is representation by a continuous number:
a quantity rᵢ of the substance i is indicated by a bit representation of a following mathematical expression 5; and
whether or not the substance i is contained in the composite material is indicated by the bit representation of the mathematical expression 5:
[Math 3] ${r}_{i} = {\sum }_{j = 1}^{{m}_{i}} {C}_{j} {q}_{ij}$ (5) where:
qᵢⱼ is a number 0 or 1 of a binary representation of a proportion or content of the substance i;
Cⱼ is a coefficient related to the binary representation of the proportion or content of the substance i;
C₁ is a minimum unit for the quantity of substances to be mixed in the composite material;
mᵢ is a maximum natural number at which Cₘ < M_{i,MAX} holds true; and
M_{i,MAX} is a maximum quantity for the quantity rᵢ of the substance i.

4. The information processing device according to any one of claims 1 to 3, wherein, when a condition regarding a quantity of a substance to be mixed in the composite material is representation by a discrete number:
a quantity rᵢ of the substance i is indicated by the bit representation; and
whether or not the substance i is contained in the composite material is indicated by the bit representation.

5. The information processing device according to any one of claims 1 to 4, wherein the mathematical expressions 1 and 2 represent constraints on the proportions or contents in the composition of the composite material specifying that the substance B is not used when the substance A is used.

6. The information processing device according to claim 2, wherein the mathematical expressions 3 and 4 represent constraints on the proportions or contents in the composition of the composite material specifying that the substance A is not used when the substance B is used.

7. An information processing system comprising:
an annealing-type optimization machine; and
an information processing device configured to assist preparation of an Ising model for allowing the annealing-type optimization machine to solve an optimal solution search problem for a composite material with constraints on proportions or contents in a composition of the composite material,
wherein the information processing device includes:
an input receiving part configured to receive as input:
an objective function that represents a property of the composite material according to the proportions or contents in the composition of the composite material; and
a constraint on the proportions or contents in the composition of the composite material; and
a data preparation part configured to prepare the Ising model that allows the annealing-type optimization machine to find an optimal solution for the proportions or contents in the composition of the composite material, the optimal solution satisfying a constraint expression that represents the constraint on the proportions or contents in the composition of the composite material and optimizing the property of the composite material,
wherein, when the composite material is a mixture of substances:
a quantity rᵢ of a substance i is indicated by a bit representation; and
whether or not the substance i is contained in the composite material is indicated by the bit representation,
wherein the constraint expression includes conditions that require that following mathematical expressions 1 and 2 be satisfied: [Math 4] $\left\{\begin{matrix}\left({m}_{B}+1\right) \left(1-{q}_{A 0}\right) + {\sum }_{j = 1}^{{m}_{i}} {q}_{Bj} \leq {m}_{B} + 1 & \left(1\right) \\ \left({m}_{B}+1\right) \left(1-{q}_{A 0}\right) + {\sum }_{j = 1}^{{m}_{i}} {q}_{Bj} \geq 1 - {q}_{B 0} & \left(2\right)\end{matrix}\right. ,$ where:
q_{A0} is an auxiliary variable that is 0 when a substance A is contained in the composite material and that is 1 when the substance A is not contained in the composite material;
q_{B0} is an auxiliary variable that is 0 when a substance B is contained in the composite material and that is 1 when the substance B is not contained in the composite material;
m_{B} is a number of bits required to represent a quantity r_{B} of the substance B to be mixed in the composite material; and
q_{Bj} is a number 0 or 1 of a binary representation of a proportion or content of the substance B in the composite material, and
wherein the constraint expression is arranged such that, when a condition included in the constraint expression fails to satisfy at least one of the mathematical expression 1 or 2, a proportion or content represented by the failing condition is excluded from the optimal solution for the composition of the composite material.

8. A program for causing an information processing device to perform steps to assist preparation of an Ising model for allowing an annealing-type optimization machine to solve an optimal solution search problem for a composite material with constraints on proportions or contents in a composition of the composite material, the steps comprising:
receiving as input:
an objective function that represents a property of the composite material according to the proportions or contents in the composition of the composite material; and
a constraint on the proportions or contents in the composition of the composite material; and
preparing the Ising model that allows the annealing-type optimization machine to find an optimal solution for the proportions or contents in the composition of the composite material, the optimal solution satisfying a constraint expression that represents the constraint on the proportions or contents in the composition of the composite material and optimizing the property of the composite material,
wherein, when the composite material is a mixture of substances:
a quantity rᵢ of a substance i is indicated by a bit representation; and
whether or not the substance i is contained in the composite material is indicated by the bit representation,
wherein the constraint expression includes conditions that require that following mathematical expressions 1 and 2 be satisfied: [Math 5] $\left\{\begin{matrix}\left({m}_{B}+1\right) \left(1-{q}_{A 0}\right) + {\sum }_{j = 1}^{{m}_{i}} {q}_{Bj} \leq {m}_{B} + 1 & \left(1\right) \\ \left({m}_{B}+1\right) \left(1-{q}_{A 0}\right) + {\sum }_{j = 1}^{{m}_{i}} {q}_{Bj} \geq 1 - {q}_{B 0} & \left(2\right)\end{matrix}\right. ,$ where:
q_{A0} is an auxiliary variable that is 0 when a substance A is contained in the composite material and that is 1 when the substance A is not contained in the composite material;
q_{B0} is an auxiliary variable that is 0 when a substance B is contained in the composite material and that is 1 when the substance B is not contained in the composite material;
m_{B} is a number of bits required to represent a quantity r_{B} of the substance B to be mixed in the composite material; and
q_{Bj} is a number 0 or 1 of a binary representation of a proportion or content of the substance B, and
wherein the constraint expression is arranged such that, when a condition included in the constraint expression fails to satisfy at least one of the mathematical expression 1 or 2, a proportion or content represented by the failing condition is excluded from the optimal solution for the composition of the composite material.

9. A method for assisting preparation of an Ising model for allowing an annealing-type optimization machine to solve an optimal solution search problem for a composite material with constraints on proportions or contents in a composition of the composite material, the method comprising:
receiving as input:
an objective function that represents a property of the composite material according to the proportions or contents in the composition of the composite material; and
a constraint on the proportions or contents in the composition of the composite material; and
preparing the Ising model that allows the annealing-type optimization machine to find an optimal solution for the proportions or contents in the composition of the composite material, the optimal solution satisfying a constraint expression that represents the constraint on the proportions or contents in the composition of the composite material and optimizing the property of the composite material,
wherein, when the composite material is a mixture of substances:
a quantity rᵢ of a substance i is indicated by a bit representation; and
whether or not the substance i is contained in the composite material is indicated by the bit representation,
wherein the constraint expression includes conditions that require that following mathematical expressions 1 and 2 be satisfied: [Math 6] $\left\{\begin{matrix}\left({m}_{B}+1\right) \left(1-{q}_{A 0}\right) + {\sum }_{j = 1}^{{m}_{i}} {q}_{Bj} \leq {m}_{B} + 1 & \left(1\right) \\ \left({m}_{B}+1\right) \left(1-{q}_{A 0}\right) + {\sum }_{j = 1}^{{m}_{i}} {q}_{Bj} \geq 1 - {q}_{B 0} & \left(2\right)\end{matrix}\right. ,$ where:
q_{A0} is an auxiliary variable that is 0 when a substance A is contained in the composite material and that is 1 when the substance A is not contained in the composite material;
q_{B0} is an auxiliary variable that is 0 when a substance B is contained in the composite material and that is 1 when the substance B is not contained in the composite material;
m_{B} is a number of bits required to represent a quantity r_{B} of the substance B to be mixed in the composite material; and
q_{Bj} is a number 0 or 1 of a binary representation of a proportion or content of the substance B, and
wherein the constraint expression is arranged such that, when a condition included in the constraint expression fails to satisfy at least one of the mathematical expression 1 or 2, a proportion or content represented by the failing condition is excluded from the optimal solution for the composition of the composite material.

10. An information processing device configured to assist preparation of an Ising model for allowing an annealing-type optimization machine to solve an optimal solution search problem for a composite material with constraints on proportions or contents in a composition of the composite material, the information processing device including:
an input receiving part configured to receive as input:
an objective function that represents a property of the composite material according to the proportions or contents in the composition of the composite material; and
a constraint on the proportions or contents in the composition of the composite material; and
a data preparation part configured to prepare the Ising model that allows the annealing-type optimization machine to find an optimal solution for the proportions or contents in the composition of the composite material, the optimal solution satisfying a constraint expression that represents the constraint on the proportions or contents in the composition of the composite material and optimizing the property of the composite material,
wherein, when the composite material is a mixture of substances:
a quantity rᵢ of a substance i is indicated by a bit representation; and
whether or not the substance i is contained in the composite material is indicated by the bit representation,
wherein the constraint expression includes conditions that require that following mathematical expressions 6, 7, and 8 be satisfied: [Math 7] $\left\{\begin{matrix}\left({m}_{i}+1\right) {q}_{i 0} + {\sum }_{j = 1}^{{m}_{i}} {q}_{ij} \leq {m}_{i} + 1 & \left(6\right) \\ \left({m}_{i}+1\right) {q}_{i 0} + {\sum }_{j = 1}^{{m}_{i}} {q}_{ij} \geq 1 & \left(7\right) \\ {\sum }_{i = 1}^{N} {q}_{i 0} \geq N - 1 & \left(8\right)\end{matrix}\right.$ where:
qᵢ₀ is an auxiliary variable that is 0 when the substance i is contained in the composite material and that is 1 when the substance i is not contained in the composite material;
mᵢ is a number of bits required to represent the quantity rᵢ of the substance i to be mixed in the composite material; and
qᵢⱼ is a number 0 or 1 of a binary representation of a proportion or content of the substance i in the composite material, and
N is a number of substances i subject to a constraint represented by the mathematical expression 8, and
wherein the constraint expression is arranged such that, when a condition included in the constraint expression fails to satisfy at least one of the mathematical expression 6, 7, or 8, a proportion or content represented by the failing condition is excluded from the optimal solution for the composition of the composite material.
